(19) Europäisches Patentamt
European Patent Office
Office européen des brevets

(11) EP 4 414 928 A1

(12) EUROPEAN PATENT APPLICATION

(43) Date of publication:
**14.08.2024 Bulletin 2024/33**

(21) Application number: **24154613.4**

(22) Date of filing: **30.01.2024**

(51) International Patent Classification (IPC):
***G06T 5/20*** (2006.01) ***G06T 5/70*** (2024.01)
***G06T 5/75*** (2024.01) ***G06T 5/60*** (2024.01)

(52) Cooperative Patent Classification (CPC):
**G06T 5/70; G06T 5/60;** G06T 2207/10081; G06T 2207/10088; G06T 2207/10132; G06T 2207/20012; G06T 2207/20084; G06T 2207/20092; G06T 2207/30096

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.02.2023 JP 2023020136**

(71) Applicant: **FUJIFILM Healthcare Corporation**
**Kashiwa-shi, Chiba 277-0804 (JP)**

(72) Inventors:
- **YOKOSAWA, Suguru**
**Chiba, 277-0804 (JP)**
- **SUZUKI, Atsuro**
**Chiba, 277-0804 (JP)**
- **SHIRAI, Toru**
**Chiba, 277-0804 (JP)**

(74) Representative: **Strehl Schübel-Hopf & Partner**
**Maximilianstrasse 54**
**80538 München (DE)**

(54) **IMAGE PROCESSING DEVICE, MEDICAL IMAGING APPARATUS INCLUDING THE SAME, AND IMAGE PROCESSING METHOD**

(57) Provided is a noise-reduced image in which noise and artifacts, which pose a problem in intraoperative MRI, are reduced for each pixel in a manner desired by a user and a preference of the user for a tissue or a site that the user wants to observe is reflected.

In a case of generating and presenting a third medical image by using a first medical image acquired by an MRI apparatus (S1) and a second medical image obtained by performing processing of reducing noise and artifacts with respect to the first medical image (S2), a difference for each pixel between the first medical image and the second medical image is taken (S3), a weighting value for each pixel is calculated using a generated difference image (S4), and a user's change for weighting is received (S5, S6, S7). The finally decided-on weighting value is used to combine the first medical image and the second medical image through weighted averaging for each pixel (S8), and the combined image is presented (S9).

FIG. 3

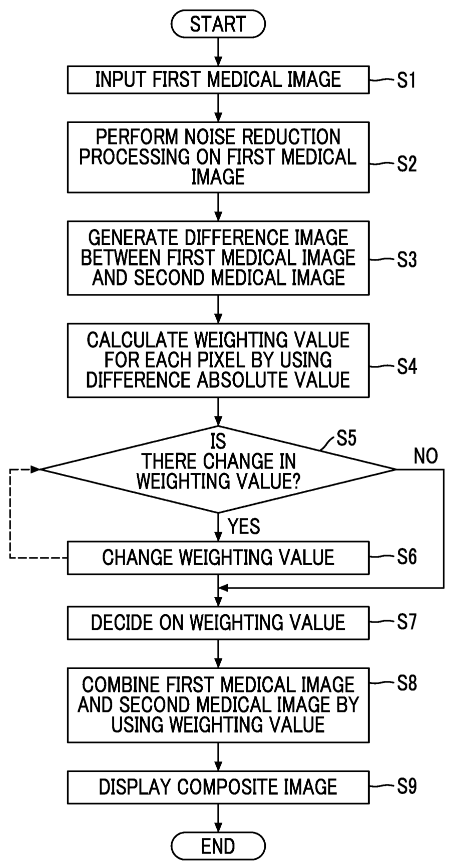


EP 4 414 928 A1

## Description

## INCORPORATION BY REFERENCE

The present application claims priority from Japanese patent application JP-2023-020136 filed on Feb 13, 2023, the content of which is hereby incorporated by reference into this application.

## BACKGROUND OF THE INVENTION

1. Field of the Invention

The present invention relates to a technique for processing an image acquired by a medical imaging apparatus, and particularly, to an image processing technique for appropriately processing noise and artifacts of a medical image to be displayed according to a situation and presenting the medical image in a case of performing intraoperative imaging.

2. Description of the Related Art

In recent years, intraoperative imaging, which allows for imaging using an image diagnostic apparatus during surgery and progressing with the surgery appropriately while checking a resection site or the like, has become widespread. As a means of intraoperative imaging, an MRI apparatus, a CT apparatus, an ultrasound imaging apparatus, and the like are utilized. By repeatedly conducting imaging through these imaging apparatuses during surgery and proceeding with the surgery while checking, for example, a tumor, it is expected to prevent the tumor from being left behind while preserving a normal tissue, and the like.

The causes of occurrence of noise and artifacts in medical images vary depending on factors such as types of imaging apparatuses, imaging conditions, and environment in which the apparatus is placed, and particularly, in intraoperative imaging, peculiar noise or artifacts may occur due to factors such as instruments used in surgery and the movement of an examination target.

For example, in the intraoperative MRI, the MRI apparatus is installed in an open-space operating room, not in a shielded room that blocks external radio wave noise. Basically, a device that is a source of noise is operated in a manner that power is turned off before conducting MRI imaging. However, due to factors such as forgetting to turn off the power or the introduction of unforeseen devices, it is currently not possible to completely prevent the occurrence of noise. Therefore, in a case where noise has occurred in the image, the basic operation is to use the image as it is in a case where the tumor is visible, and to perform re-imaging in a case where the noise is severe and the tumor is difficult to discern. Since re-imaging leads to an extension in surgical time, noise suppression in the intraoperative MRI has become a significant problem.

In MRI, various techniques for suppressing noise and artifacts occurring in images have been put into practical use, and various methods for solving side effects associated with denoising have also been proposed. For example, in WO2009/128213A, it is proposed that a noise-removed image and a signal-enhanced image are created from a captured image, and these are weighted and combined, in order to remove noise while suppressing edge blurring associated with the noise removal. In addition, in JP2020-119429A, it is proposed that an optimal value of denoising strength is determined based on a plurality of denoised images created with varying levels of the denoising strength, and a difference image between the plurality of denoised images and an original image, thereby improving denoising accuracy. Further, some techniques for applying CNN or the like that have learned various noise patterns to noise removal have also been proposed.

## SUMMARY OF THE INVENTION

With the above-described related arts, it is possible to improve image quality while achieving both noise suppression and image blur suppression. However, in the conventional methods, since weights for combining images, denoising strength, and the like are common throughout the entire image, it is not possible to perform different image quality improvements for each region. In particular, in an intraoperative image, noise and artifacts occur only in a specific region. Therefore, there is a demand to suppress noise and artifacts in an occurrence region while suppressing unnecessary changes in image quality caused by processing or maintaining the original image from the viewpoint of reliability in the other region.

Further, since an operating surgeon who uses the intraoperative image reads various information from the image, an image to which a noise suppression image processing method is applied may not necessarily be the most appropriate image for the operating surgeon. Some requests are dependent on preferences. For example, when recognizing margins of the tumor, some individuals may find it easier to recognize the margins with a bit of noise remaining.

An object of the present invention is to solve these problems and to present, particularly for a medical image captured during surgery, an image in which noise and artifacts that may occur during the surgery are effectively suppressed according to a preference of an operator.

In order to solve the above-described problems, the present invention provides an image processing device comprising a unit that accepts a request for noise reduction of an operator such as an operating surgeon and a unit that performs noise reduction processing specialized to a particular region, in processing of the medical image. In the processing specialized to the region, a weighting value in a case of using a difference between an original image, which is acquired by an imaging apparatus, and an image, which is obtained by performing general noise reduction processing on the original image, to perform weighted addition of both the images is decided on for each pixel, and both the images are combined.

That is, according to an aspect of the present invention, there is provided an image processing device that generates and presents a third medical image by using a first medical image acquired by a medical imaging apparatus and a second medical image obtained by performing processing of reducing noise and artifacts with respect to the first medical image, the image processing device comprising: a difference image generation section that takes a difference for each pixel between the first medical image and the second medical image and generates a difference image; a weight calculation section that calculates a weighting value for each pixel by using the difference image; a weight decision section that receives a user instruction including a change of the weighting value for each pixel and decides on a final weighting value; and a composite image generation section that uses the weighting value decided on by the weight decision section to combine the first medical image and the second medical image through weighted averaging for each pixel.

In addition, according to another aspect of the present invention, there is provided an imaging apparatus comprising a function of the image processing device of the aspect of the present invention as a function of an image processing unit.

Further, according to still another aspect of the present invention, there is provided an image processing method of generating and presenting a third medical image by using a first medical image acquired by a medical imaging apparatus and a second medical image obtained by performing processing of reducing noise and artifacts with respect to the first medical image, the image processing method comprising: taking a difference for each pixel between the first medical image and the second medical image and generating a difference image; calculating a weighting value for each pixel by using the difference image; receiving a user instruction including a change of the weighting value for each pixel and deciding on a final weighting value; and using the decided-on weighting value to combine the first medical image and the second medical image through weighted averaging for each pixel.

It should be noted that, in the present invention, a target of image processing includes noise and artifacts caused by various causes, but these are collectively referred to simply as noise in the present specification. Similarly, a region where noise and artifacts have occurred on an image is simply referred to as a noise occurrence region.

According to the aspects of the present invention, in a case where an original image and an image processed by general noise reduction processing are combined through weighted addition, use of a weight that corresponds to a degree of noise occurrence, which is calculated for each pixel, and that reflects a user's instruction makes it possible to obtain an image in which an influence of potential noise, unexpectedly occurring noise, or the like is eliminated without compromising information of the original image. In particular, it is possible to perform processing that reflects a preference of an operating surgeon or the like for a region that the operating surgeon or the like wants to observe. As a result, it is possible to provide an image in which noise is reduced in a manner desired by the user for a tissue, which is a target of surgery, or a surrounding tissue, and it is possible to reduce a probability of re-imaging or extensions in surgical time associated with the re-imaging.

## BRIEF DESCRIPTION OF THE DRAWINGS

Fig. 1 is a diagram showing an outline of an examination system including an imaging apparatus and an image processing device.

Fig. 2 is a functional block diagram of the image processing device according to Embodiment 1 of the present invention.

Fig. 3 is a diagram showing a flow of processing of the image processing device of Embodiment 1.

Fig. 4 is a diagram showing one embodiment of a noise reduction section.

Fig. 5 is a diagram illustrating a weighting value.

Fig. 6A is a diagram showing an example of a GUI that receives a user's change.

Fig. 6B is a diagram showing another example of the GUI that receives the user's change.

Fig. 7 is a diagram showing details of the processing of Fig. 3.

Fig. 8 is a diagram illustrating processing of the embodiment of the present invention.
Fig. 9 is a functional block diagram of an image processing device of Embodiment 2.
Fig. 10 is a diagram showing an example of a flow of processing of the image processing device of Embodiment 2.
Fig. 11 is a diagram showing another example of the flow of the processing of the image processing device of Embodiment 2.
Fig. 12 is a diagram illustrating region specification in a data space.
Fig. 13 is a diagram showing an example of a noise pattern.
Fig. 14 is a diagram showing an example of a weight calculation section of Embodiment 3.
Fig. 15 is a diagram showing another example of the weight calculation section of Embodiment 3.

## DESCRIPTION OF THE PREFERRED EMBODIMENTS

Hereinafter, embodiments of an image processing device and an image processing method according to the present invention will be described with reference to the drawings.

The image processing device of the embodiment of the present invention is an image processing device that is used to process a medical image acquired by a medical imaging apparatus to present the processed image to a doctor, an imaging technician, or the like who is performing surgery or examinations (hereinafter, collectively referred to as a user) as an image useful for an intraoperative image, and is configured to present the image after processing to, for example, a display device 30A placed in a room in which a medical imaging apparatus (hereinafter, simply referred to as an imaging apparatus) 20 is installed, as shown in Fig. 1. An image processing device 10 itself may be placed in a separate operation room or at a remote location from the examination room, may be located in the same examination room as the imaging apparatus 20, or may be an accessory device of the imaging apparatus itself.

The imaging apparatus 20 is not particularly limited as long as it is an apparatus that can be used for intraoperative imaging, and examples thereof include an MRI apparatus, a CT apparatus, and an ultrasound imaging apparatus. In Fig. 1, as an example, an MRI apparatus provided with a static magnetic field generation magnet 201, a gradient magnetic field coil 202, and high-frequency coils (a transmission RF coil 203 and a reception RF coil 204) is shown as the medical imaging apparatus 20. In the MRI apparatus, a gradient magnetic field power supply 212, a transmitter 213, and a receiver 214, to which the gradient magnetic field coil 202 and the high-frequency coils are connected, are operated by a sequencer 215, a nuclear magnetic resonance signal generated by a subject 205 is received, and an image of the subject 205 is reconstructed using the nuclear magnetic resonance signal.

The imaging apparatus 20 represented by such an MRI apparatus comprises a computer 220 that performs imaging control and computational operations such as image reconstruction and image processing. The image processing performed by the computer 220 may include, in addition to image reconstruction using general Fourier transformation and sequential reconstruction, known noise reduction processing on the reconstructed image, and the like.

Further, the display device 30A that displays the reconstructed image, a user interface (UI) unit 50 that is provided with an input device for the user to input commands or data, a display device which displays GUI, and the like, an external storage device (not shown), and the like are connected to the computer 220, the user can send instructions necessary for the operation of the imaging apparatus 20 or transmit images to the external storage device, via the UI unit 50. The display device 30A provided in the imaging apparatus 20 can also function as a display device that displays a processing result of the image processing device 10.

An image obtained by such an MRI apparatus is susceptible to an influence of external electromagnetic waves and is prone to noise and artifacts caused by, for example, electromagnetic waves or the like emitted from instruments used during surgery. In addition, in an imaging apparatus other than the MRI apparatus, in addition to general Gaussian noise and noise corresponding to the characteristics of the modality, artifacts may occur due to irregular movements, position changes, or the like of the subject during surgery.

The image processing device 10 processes a subject image (original image) including noise or artifacts, which is captured by the imaging apparatus 20, and generates an image in which noise or artifacts are appropriately reduced and image quality expectations of an operator for a region that the operator needs to observe are satisfied. The function of the image processing device 10 can be constructed in a general-purpose computer provided with a CPU and a memory. Although the image processing device 10 is shown as a device independent of the imaging apparatus 20 in Fig. 1, the image processing device 10 may be constructed in the computer 220 of the imaging apparatus 20.

Hereinafter, an outline of the configuration and operation of the image processing device 10 will be described by using a case where the image processing device 10 is provided independently of the computer of the imaging apparatus 20 as an example.

As shown in Figs. 1 and 2, the image processing device 10 comprises a third image generation unit 110 that generates an image useful for the user who is operating the imaging apparatus 20, and a display controller 130 that generates an image for presenting the image generated by the third image generation unit 110 and that displays the generated image on the display device. Although not shown in Fig. 1, the image processing device 10 also includes a memory or a storage device that stores various kinds of data used by the third image generation unit 110, image data during calculation, and the like. Further, a function other than the third image generation unit 110 need not be provided, a part of the processing performed by the image processing device 10 may be performed by a programmable IC such as ASIC, and the image processing device 10 may include an AI function such as CNN that has been trained on the computer inside or outside of the image processing device 10.

The image processing device 10 is connected to the UI unit 50 provided with a display device 30 that displays an image as a processing result and an input device 40 that receives an instruction from the user. In a case where the imaging apparatus 20 comprises a user interface having the same function (Fig. 1: UI unit 50), the user interface 50 can also be used as the UI unit 50 of the image processing device 10. The input device 40 may be an independent device that the user can operate separately from the operation of the imaging apparatus 20 during surgery, or may be incorporated into a console of the image processing device or the imaging apparatus. Further, as shown in Fig. 1, in a case where the imaging apparatus 20 comprises the display device 30A separately from the console, the processing result image of the image processing device 10 may be displayed on the display device 30A.

The third image generation unit 110 uses an original image (first medical image), which is reconstructed by the imaging apparatus 20, and a noise-reduced image (second medical image), which is obtained by performing known noise reduction processing on the first medical image, to generate a third image in which noise is removed, with noise and artifacts occurring during imaging or during surgery performed during imaging as a target, and presents the third image to the user.

With regard to the second medical image, in a case where noise reduction processing is included as image processing performed by the computer 220 of the imaging apparatus 20, the image processed by the computer 220 may be used as the second medical image, or the image processing device 10 may import the original image from the imaging apparatus 20 and perform known noise reduction processing.

As shown in Fig. 2, the third image generation unit 110 comprises an image reception section 111 that receives an image from the imaging apparatus 20, a noise reduction processing section 112, a difference image generation section 113 that generates a difference image between the first medical image and the second medical image, a weight calculation section 114 that uses a pixel value of each pixel in the difference image to calculate a weighting value for each pixel, a weight decision section 115 that receives user designation for the weighting value calculated by the weight calculation section 114 and that changes the weighting value as necessary, and an image combining section 116 that uses the finally decided-on weighting value to combine the images. A dashed rectangle indicating the noise reduction processing section 112 means that this function may be either a function of the image processing device 10 or a function of the imaging apparatus 20, as described above.

Next, Fig. 3 shows a flow of the operation of the third image generation unit 110 having the above-described configuration. Here, as an example, it is assumed that the imaging apparatus 20 is an MRI apparatus 20A and that intraoperative MRI is performed.

First, in a case where an image (first medical image) reconstructed by the MRI apparatus 20A is input by the image reception section 111 (S 1), the noise reduction processing section 112 performs noise reduction processing on the first medical image (S2). The noise reduction processing performed here is not particularly limited as long as it is generally known processing, and for example, any of processing of removing artifacts characteristically occurring depending on an imaging method through computational operations, processing using a filter, processing using CNN that has learned a noise image and a noise-free image as learning data, or the like can be employed, and a plurality of the processing may also be combined as necessary. Further, in the noise reduction processing using the filter, the filtering strength may be adjusted according to the noise, and the sharpness of the image after noise reduction may be adjusted. Through the processing by the noise reduction processing section 112, an image (second medical image) in which noise is reduced throughout the entire image can be obtained.

Next, the difference image generation section 113 takes a difference between the original image before the noise reduction and the second medical image after the noise reduction processing to generate a difference image and to calculate an absolute value of the difference of each pixel (S3).

The weight calculation section 114 calculates a weighting value for each pixel by using the absolute value of the difference of each pixel calculated by the difference image generation section 113 (S4). The weighting value is calculated such that, in a region of a pixel where the difference (absolute value) between the original image and the image after noise reduction is large, which is considered that significant noise has occurred, a weight of the noise-reduced image is increased, while in a region with a small difference, a weight of the original image is increased.

A specific calculation method will be described below, but for example, it is calculated using a weight coefficient and a value (fixed value) that is empirically determined in advance to be optimal according to noise characteristics such as a noise pattern, and in step S4, the fixed value and the weight coefficient are set in advance to predetermined reference values, and these are used to calculate the weighting value.

Next, the user's change for the weighting value is received (S5). In order to receive the user's change, the display device of the UI unit 50 presents the calculated weighting value or the image combined with the weighting value.

The user checks the presented weighting value or the provisionally combined image, determines whether or not the weighting value needs to be changed, and changes the weighting value by using the GUI displayed on the display device of the UI unit in a case where the change is necessary. In a case where no change is necessary, the weighting value calculated using the GUI in the same manner is finally confirmed. As a result, the weighting value to be used in the subsequent combining processing is decided on (S7).

The image combining section 116 combines the original image and the noise-reduced image for each pixel by using the weighting value for each pixel to generate a composite image which is the third image (S8). The processing of the third image generation unit 110 is completed with the above S1 to S8.

The display controller 130 causes the display device 30 to display the third image generated by the third image generation unit 110 (S6). The form of the display is not particularly limited and will be described in detail in the embodiment to be described below. However, in order to make it easier for the user to check a region where processing is performed with respect to the original image, particularly a region with a high degree of noise reduction processing, it is preferable to display two images in parallel or superimposed. In a case of superimposing the two images, color coding or the like is performed to enhance visibility.

According to the present embodiment, the magnitude of the difference (absolute value) between the original image and the image after noise reduction is obtained for each pixel, and the weight for each pixel is set based on the magnitude of the difference to combine the images, whereby it is possible to present a third medical image in which noise reduction focused on noise that interferes with the checking of the original image is performed while preserving the maximum amount of information from the original image. As a result, it is possible to appropriately suppress the influence of noise that occurs temporarily and spatially in a limited manner due to operations of unforeseen devices during surgery, or the like and there is no risk that sites such as a critical site, which is a target of the surgery, are blurred by noise reduction throughout the entire image, which makes it possible to provide a useful intraoperative image.

Further, in deciding on the weighting, a configuration is employed in which the weighting is calculated on the basis that the weight of the noise-reduced image is increased in a region with significant noise and the weight of the original image is increased in a region with low noise, and the weighting is changeable in accordance with the preference of the user, so that it is possible for the user to fulfill requirements, such as referring to the original image even in a case where there is noise or checking a noise-reduced state, and the effect of user support in intraoperative imaging can be enhanced.

Although the outline of the image processing device 10 of the embodiment of the present invention has been described above, the details of the processing performed by the image processing device 10 will be further described in the following embodiments.

Embodiment 1

In the present embodiment, the weight calculation section 114 of the third image generation unit 110 calculates a weight coefficient $\alpha$ for each pixel by using the absolute value of the difference of each pixel calculated by the difference image generation section 113 and calculates a weighting value using the weight coefficient $\alpha$ as an exponent, as the weighting value.

Since the flow of processing is the same as that of Fig. 3, the processing of the present embodiment will be described below with reference to Fig. 3.

First, the noise reduction processing section 112 performs noise reduction processing on the original image (first medical image) acquired by the MRI apparatus 20A and generates the second medical image (S1, S2). Here, as an example, noise removal is performed using a super resolution convolutional neural network (SRCNN) of all three layers as shown in Fig. 4. This CNN is designed to remove random noise by using a random noise pattern as learning data, and the image after noise reduction (second medical image) tends to become blurred, which causes brain sulci and tumor boundaries to become indistinct, but the influence of the image processing cannot be grasped.

The third image generation unit 110 combines the original image and the CNN-processed image to obtain the combined third image. In that case, a composite image in which information that has been compromised by the noise reduction processing is reproduced as faithfully as possible is generated such that the combined image tends to lean toward the image after CNN processing for a location where local noise or artifacts have occurred, while the combined image leans toward the original image for the other positions.

Therefore, first, the difference image generation section 113 calculates an absolute value d of the difference in signal intensity for each pixel between the original image and the image after CNN processing by using Equation (1) (S3).

$$d(i,j) = \left|I_{CNN}(i,j) - I_{org}(i,j)\right| \tag{1}$$

In Equation (1), $I_{CNN}$ represents the image after CNN processing, $I_{org}$ represents the original image, and (i,j) represents a pixel position (hereinafter, the same).

The difference in signal intensity, which is denoted by d, is large in a position where local noise or artifacts have occurred, but is small in the other region.

The weight calculation section 114 calculates the coefficient $\alpha$ (referred to as a weight coefficient) in which the absolute value d of the difference is standardized by the average value of d by using Equation (2). The weight coefficient $\alpha$ is obtained for each pixel and is used to decide on the weighting value. In the present embodiment, a weight of an image in a case of performing weighted addition of the original image and the image after CNN processing is set as a fixed value (fixed weight) W, and the weight coefficient $\alpha$ is used as an exponent of the fixed weight. Therefore, a lower limit value and an upper limit value are set in advance for $\alpha$. As an example, it is assumed that the lower limit value is 0.01 and the upper limit value is 100.

$$\alpha(i,j) = \frac{\bar{d}}{d(i,j)} \qquad (2)$$

Next, the weight calculation section 114 decides on the weighting value $W^{\alpha}$ in the image weighted addition represented by Equation (3) (S4), and the image combining section 116 uses Equation (3) to generate an image (third image) in which a degree of noise processing is adjusted according to a noise occurrence position.

$$I_{adj}(i,j) = \left(I_{CNN}(i,j) - I_{org}(i,j)\right) \cdot w^{\alpha(i,j)} + I_{org}(i,j) \qquad (3)$$

Here, $I_{adj}$ represents an image after adjustment, and W represents the fixed weight. The fixed weight is a value in a range of 0 to 1 and is a value adjusted according to a noise pattern or the like, but in the present embodiment, the fixed weight is set to a reference value as a default, for example, 0.5, and then is used as a variable that is changeable by the user.

A relationship between such a fixed weight W and the weighting value $W^{\alpha}$ using the weight coefficient for each pixel as an exponent is schematically shown on the upper side of Fig. 5. As shown in a graph on the upper side of Fig. 5, in a case where $\alpha$, which is an indicator of the pixel signal intensity difference, satisfies $\alpha$ = 1 (a straight line), W" takes the same value as W, but exponentially changes based on the value of $\alpha$. A graph on the lower side of Fig. 5 shows a case where W = 0.5 as an example, and as shown in this graph, in a case where $\alpha$ is smaller than 1, the weighting value $W^{\alpha}$ takes a value greater than W, which results in a larger weight for the CNN-processed image. That is, the weighting value for the noise occurrence position where the absolute value of the difference is large (the weight coefficient $\alpha$ is small) is decided on to be large.

In Equation (3), although the weight coefficient is used as the exponent of the fixed weight W, the method of deciding on the weighting value using the weight coefficient $\alpha$ for each pixel is not limited to Equations (2) and (3), and for example, a method of setting a weight coefficient standardized by the maximum value of d to $\alpha'$ and using a weighting value $W\alpha'$ can also be employed.

In a case where the weighting value is calculated in this manner, the UI unit 50 displays the GUI that receives the user designation for the weighting value. The GUI may be a graph as shown on the upper side of Fig. 5 (Fig. 6A). In that case, as shown in Fig. 6A, the GUI having a form of a line 601 at the center of the graph can be made movable by the user, and the fixed value W can be changed by moving the line 601 parallel to the left side or the right side. In that case, it is preferable to provide a display on the graph such that it can be seen that moving to the right side increases the weight of the noise-reduced image in a region with significant noise, while moving to the left side increases the weight of the original image in a region with low noise. This enables the user to easily perform GUI operations that match the user's intentions. In addition, the display may include a graph (on the lower side of Fig. 6A) of the weight curve at the selected W.

Additionally, as another method, a provisionally combined image may be displayed on the display device of the UI unit 50 by performing weighted addition of the original image and the CNN-processed image using the weighting value calculated by the image combining section 116 in step S4. In this display, as shown in Fig. 6B, a configuration is employed in which a display bar 603 (GUI) indicating the weighting value or the fixed weight is displayed together with the display of a provisional composite image 602, and the user operates the display bar 603 to change the weight. As the weights change, the image combining section 116 performs addition using the changed weights and updates the composite image. The original image or the CNN-processed image may be displayed together with the composite image 602, and the user can use the images as references in a case of changing the weight.

In a case where the composite image is displayed, a setting for an ROI may be received on a display screen so that the weight may be made changeable only for the ROI. This enables the user to have an enhanced degree of freedom in combination, such as switching only an observed location to, for example, the original image (setting the weight of the noise-reduced image to zero).

In this method, for example, steps S4 to S7 of Fig. 3 may be changed as shown in Fig. 7, and changing the weighting and updating the composite image may be repeated. As a result, the user can finally obtain the composite image desired by the user.

The user may designate not only the "fixed weight W" but also the coefficient $\alpha$, or the weight itself determined by W and $\alpha$ (for example, W"), and the designation method is not limited to the GUI shown in Fig. 6A or 6B, and various methods can also be employed for designating numerical values or designating qualitative characteristics (such as the magnitude of noise reduction strength).

After the weight is finally determined, the image combining section 116 performs the weighted addition of the original image and the CNN-processed image in accordance with Equation (3) to generate a composite image which is the third image (Fig. 3: S8). In the composite image, in a case where the fixed weight is used as a reference, in pixels in which noise has occurred and the signal intensity difference is large, the CNN-processed image has a larger weight, but in pixels in which no noise has occurred, the original image has a larger weight, which results in an image that reflects more information of the original image.

In particular, by receiving the user instruction, it is possible to display an image that reflects the preference of the user.

Fig. 8 shows an example of an image generated through the image processing of the present embodiment. In the figure, the left side is the original image (image before CNN processing), the center is the image after noise reduction processing (image after CNN processing), and the right side is a composite image (adjusted image).

The original image has noise occurring in regions 801 and 802 surrounded by rectangles in the figure, and the noise disappears in the image after CNN processing by performing the noise reduction processing on this image, but the overall sharpness has decreased, which results in a slight blurring of brain sulci, bleeding parts, or the like. In the adjusted image obtained by combining these two images with an appropriate weight for each pixel using the method of the present embodiment, noise is reduced in the noise occurrence region, and sharpness close to that of the original image is obtained in the other region.

In a case of intraoperative MRI, the image (adjusted image) combined by the image combining section 116 is immediately displayed on the display device 30 disposed close to the imaging unit of the MRI apparatus. Therefore, the display controller 130 receives the composite image from the image combining section 116 and generates a display image. The display image may be only the adjusted image as shown on the right side of Fig. 8, but in order to make it easier for the user to check the adjusted region, the original image and the image after CNN processing may be displayed together, or the difference image may be displayed. As one aspect of displaying the difference image, for example, the adjusted image may be displayed in one color such as black and white, and the difference image may be superimposed and displayed on the adjusted image in a color different from that of the adjusted image. In this case, it is preferable to make the display of the difference image transparent to maintain the visibility of the adjusted image. In addition, the display of the difference image may be made userselectable between being displayed and hidden.

This enables the user to proceed with the surgery while checking and reading reliable image information.

According to the present embodiment, it is possible to provide an image in which the influence of noise is reduced while ensuring the sharpness of the tissue that the user wants to observe and reflecting the preference of the user for the region where noise has occurred even in a case where noise has occurred in an image due to potential noise or sudden radio wave noise.

Embodiment 2

In Embodiment 1, the weight is decided on by determining whether or not noise has occurred from the signal intensity difference for each pixel. However, the present embodiment is characterized by specifying a region where noise has occurred based on the signal intensity difference for each pixel and varying weighting rules between the specified region and the other region. The "noise occurrence region" in the present embodiment includes not only a region in an image space but also a region in a data space representing the magnitude of the difference.

Therefore, in the image processing device of the present embodiment, as shown in the functional block diagram of Fig. 9, the third image generation unit 110 comprises a region specification section 117. In the embodiment shown in Fig. 9, the region specification section 117 receives a user designation through the UI unit 50, which is attached to the imaging apparatus 20, and specifies the region. In Fig. 9, the same elements as those in Fig. 2 are designated by the same reference numerals, and the overlapping description will not be repeated.

Hereinafter, processing by the image processing device of the present embodiment will be described with reference to Fig. 10, with a focus on the differences from Embodiment 1.

First, in the same manner as in Embodiment 1, the original image reconstructed by the imaging apparatus 20 is input, the noise reduction processing is performed on the original image, and the difference for each pixel between the original image and the noise-reduced image is calculated (S1 to S3). The display controller 130 displays the difference image obtained using the difference for each pixel or the original image on the display device 30 of the UI unit 50 (S31).

The user looks at the image displayed on the display device 30 and designates a region where noise or artifacts have occurred. For example, in a case where the original image as shown on the left side of Fig. 8 is displayed, the regions 801 and 802 determined by the user to be noise occurrence regions are selected and designated through the input device 40 such as a pointer or a mouse. The region specification section 117 receives the user designation (S32) and specifies the noise occurrence (the region of the image space) region. Similarly, in a case where the difference image is displayed, the noise occurrence region can be specified by the user through the designation of a region with high signal intensity in the difference image via the UI unit 50.

Further, as shown in Fig. 11, a predetermined threshold value is set in advance for the weight coefficient $\alpha$ using the absolute value of the difference or Equation (2) mentioned above, and the region specification section 117 may specify a position where the weight coefficient $\alpha$ is equal to or less than the threshold value, that is, the absolute value of the difference increases, as the noise occurrence region (S33). In this case, steps S31 and S32 of Fig. 10 can be omitted. For example, in the histogram of the weight coefficient $\alpha$ as shown in Fig. 12, a region (a region on the data space) having a threshold value TH or less is set as a noise occurrence region. The threshold value can be determined by using a discriminant analysis method or the like. Alternatively, the threshold value may be set in advance, but GUI that receives a numerical value input or an input by the operation on the histogram may be displayed on the display device 30 of the UI unit 50. This makes it possible to specify the noise region based on the user's perception.

In addition, in the specification of the noise occurrence region, a method using the user designation (Fig. 10) and a method using the threshold value (Fig. 11) have been described, but any one of these methods may be employed, or both of them can also be employed. In that case, the region specification section 117 takes AND or OR on the regions designated by the two methods to specify the noise occurrence region.

After the noise region is specified, the weight calculation section 114 decides on the weighting values for the noise occurrence region and the other region in accordance with different weighting rules, respectively (S81).

An example of the method of varying weighting rules is, as shown in Fig. 10, to vary the fixed weight W used in Equation (3) between the noise occurrence region and the other region. For example, the noise occurrence region is set to have a larger fixed weight than the other region. The calculation of the actually applied weighting value $W^{\alpha}$ as a function of the weight coefficient $\alpha$ for each pixel is the same as that of Embodiment 1. However, due to the difference in the fixed weights W, in the noise occurrence region, the weighting value is greater than in the other region, and the combined image closely resembles the noise reduction processed image in the noise occurrence region.

As the method of varying the weighting rules, a weighting value based on the weight coefficient may be calculated only for the noise occurrence region, and the weighting value = the fixed weight W may be set for the other region. In this case, after the noise occurrence region is specified through the user designation, the weight coefficient need only be calculated only for that region, so that the computational load can be reduced and the time required for the presentation of the composite image can be accelerated.

After calculating the weighting value for each region, a change by the user is received, and the weighting value is changed in a case where there is a change (S5 to S7). The method of receiving the change by the user is the same as that of Embodiment 1, but in the present embodiment, a configuration may also be employed in which only the noise occurrence region received in step S32 or only the noise occurrence region specified in step S33 is changed, or after the user designates an ROI, a change may be received for the ROI.

Obtaining the composite image through the weighted addition after decision (S81), and displaying the composite image (S9) are the same as in Embodiment 1. The aspect of display can also be made the same as the aspect described in Embodiment 1. However, in a case where the threshold value of the difference between images is set at the time of region specification or in a case where the threshold value of the difference is set by the user when superimposing and displaying the difference image on the original image in a different color, the color need not be displayed for a region where the difference is lower than the threshold value. As a result, it is possible to present only the information on the region or the position that the user wants to check without presenting redundant information. In addition, in a case where two or more regions are specified by the region specification section 117, each region may be displayed with a different color. For example, a predetermined range designated by the user, a dot-like noise position selected by using the threshold value, and the like are displayed in different colors. This makes it possible to check the difference in the pattern or in the occurrence position of the noise having different occurrence causes.

Embodiment 3

In the present embodiment, image adjustment corresponding to the noise or artifact pattern is performed (the first medical image and the second medical image are combined). The functional block diagram of the image processing device of the present embodiment is basically the same as the functional block diagram of Embodiment 1 shown in Fig. 2 or the functional block diagram of Embodiment 2 shown in Fig. 7, and in the following description, reference is made to these drawings. However, the present embodiment is characterized in that the weight calculation section 114 calculates or decides on weighting values corresponding to various noise patterns.

Fig. 13 shows some patterns of noise occurring in the MR image. In Fig. 13, the upper left shows a dot-like pattern with dot-like noise occurring at unspecified positions, with other patterns representing noise occurring within a limited range, the upper right shows a zipper-like pattern with noise occurring in a dashed line shape, the lower left shows a wavy pattern in which one or a plurality of regions become streaked, and the lower right shows a mosaic-like pattern resulting in a roughened image. In addition, there is a noise pattern peculiar to the MR image. Further, the distribution of noise intensity (the pattern of the histogram) also differs depending on these noise patterns, and appropriate weighting values and fixed weights differ.

The weight calculation section 114 applies a weighting method corresponding to the noise pattern by using a processor, such as machine learning, to decide on the weighting value for each pixel.

As the processor, a known algorithm that has been developed for machine learning can be used. Hereinafter, an example of a method of deciding on the weighting value corresponding to the noise pattern will be described.

In a first method (Method 1), as shown in Fig. 14, a processor 1141 that receives the difference image as an input and outputs a noise pattern is provided, and various predetermined noise patterns and calculation algorithms for weighting values are stored in a database (DB 1142) in association with each other. The difference image represents a difference in signal intensity for each pixel between the original image and the image after noise reduction processing and includes information such as a magnitude of the signal intensity, a distribution in the image space, an intensity distribution, or the like. The processor is trained in advance using pairs of noise patterns and various difference images having these elements different from each other as training data and is trained to classify the difference images into the noise patterns.

Meanwhile, the relationship between the noise pattern and the calculation algorithm for the weighting value is specifically a calculation expression for calculating the weighting value from the weight coefficient $\alpha$ and the fixed weight W corresponding to the noise pattern. For example, a relational expression is used in which the value of the fixed weight of the image after noise reduction processing is reduced in a case where the noise intensity obtained from the difference image is relatively small, a weighting algorithm that uses the weight coefficient $\alpha$ as the exponent of the fixed weight is used in a case where the noise intensity distribution exponentially changes, and for a pattern with a noise intensity distribution having a peak, heavier weights are set only in pixels near the peak. Although the DB 1142 that stores the relationship between such a noise pattern and the calculation algorithm for the weighting value is provided in the weight calculation section 114 in Fig. 14, the DB 1142 may be provided in the storage device in the image processing device 10 or the external storage device.

In a case where the processor receives the difference image as an input and outputs the noise pattern corresponding to the difference image, the weight calculation section 114 refers to the DB to select a calculation algorithm corresponding to the output noise pattern and calculates the weighting value for each pixel.

In a second method (Method 2) using a processor, as shown in Fig. 15, a processor 1143 that receives the difference image created from the difference between the first medical image and the second medical image as an input and outputs the weighting value for each pixel is used. The processor 1143 is configured with, for example, a machine learning algorithm that has learned to output the optimal fixed weight and the relationship between the fixed weight and the weighting value by using, as learning data, various difference images or histograms thereof, the weighting value for each pixel, which is adjusted in advance, or the weighting value to be decided on according to the noise pattern, which is obtained by classifying the noise patterns through Method 1 and storing the noise patterns in the database.

In Method 1, the processor 1141 first classifies the difference image into a noise pattern and then refers to the DB 1142 in which the noise pattern and the calculation algorithm for the weighting value are associated with each other, but in the present method, the processor 1143 receives the difference image as an input and outputs the optimal weighting value without outputting the noise pattern.

In both Methods 1 and 2, further receiving the change of the weighting value by the user after deciding on the weighting value, combining images by using the finally decided-on weighting value through the image combining section 116, and displaying the combined image on the display device 30 are the same as in Embodiment 1 and Embodiment 2, and the form of the display is also the same.

According to the present embodiment, it is possible to decide on the optimal weighting value according to the difference image through the trained CNN or the like, and it is possible to present the adjusted image with high accuracy corresponding to the noise pattern.

Explanation of References

10: image processing device
110: third image generation unit
111: image reception section
112: noise reduction processing section
113: difference image generation section
114: weight calculation section
115: weight decision section
116: image combining section
117: region specification section
20: imaging apparatus
20A: MRI apparatus
30: display device
30A: display device
40: input device
50: UI unit

## Claims

1. An image processing device that generates and presents a third medical image by using a first medical image acquired by a medical imaging apparatus and a second medical image obtained by performing processing of reducing noise and artifacts with respect to the first medical image, the image processing device comprising one or more processors configured to:

   take a difference for each pixel between the first medical image and the second medical image and generates a difference image;
   calculate a weighting value for each pixel by using the difference image;
   receive a user instruction including a change of the weighting value for each pixel and decides on a final weighting value; and
   use the weighting value decided on by the weight decision section to combine the first medical image and the second medical image through weighted averaging for each pixel.

2. The image processing device according to claim 1, wherein the one or more processors include:
   a noise reduction section that generates the second medical image in which noise and artifacts are reduced with respect to the first medical image.

3. The image processing device according to claim 1, wherein the one or more processors include:
   a region specification section that uses the difference image to specify a region where noise and artifacts have occurred in the first medical image.

4. The image processing device according to claim 3,
   wherein the one or more processors vary a conditional expression used to calculate the weighting value between the region specified by the region specification section and the other region.

5. The image processing device according to claim 3,
   wherein the one or more processors calculate the weighting value such that a weight of a pixel of the second medical image is greater than a weight of a pixel of the first medical image for the region specified by the region specification section, and a weight of a pixel of the first medical image is greater than a weight of a pixel of the second medical image for a region other than the region specified by the region specification section.

6. The image processing device according to claim 3,
   wherein the region specification section specifies the region where noise and artifacts have occurred based on a threshold value for a difference of pixel values calculated by the one or more processors.

**7.** The image processing device according to claim 3, further comprising:

a UI unit that receives a user's designation for a region where noise and artifacts have occurred,
wherein the region specification section specifies the region designated by the user via the UI unit as the region where noise and artifacts have occurred.

**8.** The image processing device according to claim 1, further comprising:

a display controller that controls an image to be displayed on a display device,
wherein the display controller displays a composite image generated by the one or more processors, and the difference image or a part of the difference image, on the display device.

**9.** The image processing device according to claim 8,
wherein the display controller displays the composite image with a first color scale, and superimposes and displays the difference image or the part of the difference image with a second color scale that is different from the first color scale, on the display device.

**10.** The image processing device according to claim 8,
wherein the display controller displays a pixel of the difference image whose pixel value is equal to or greater than a predetermined threshold value on the display device.

**11.** The image processing device according to claim 1,
wherein the one or more processors calculate a weight coefficient $\alpha$ with respect to a pixel value by using the difference image and calculates the weighting value for each pixel by using a fixed weight W (W = 0 to 1) and the weight coefficient.

**12.** The image processing device according to claim 11,
wherein the one or more processors use the weight coefficient $\alpha$ as an exponent of the fixed weight W to decide on the weighting value for each pixel, which is denoted by $W^{\alpha}$.

**13.** The image processing device according to claim 11,
wherein the one or more processors include a region specification section that uses the difference image to specify a region where noise and artifacts have occurred in the first medical image, and varies the fixed weight W according to the region where noise and artifacts have occurred and the other region.

**14.** The image processing device according to claim 11,
wherein the one or more processors include a processor that receives the difference image as an input and that outputs a weighting value for each pixel corresponding to a noise pattern.

**15.** The image processing device according to claim 1,
wherein the one or more processors include a processor that receives the difference image as an input and that outputs a noise pattern, and calculates the weighting value for each pixel by selecting, based on a correspondence between various predetermined noise patterns and calculation algorithms for weighting values, a calculation algorithm corresponding to the noise pattern output by the processor.

**16.** A medical imaging apparatus comprising:

an imaging unit that acquires a medical image of a subject; and
an image processing unit that processes the medical image acquired by the imaging unit,
wherein the image processing unit includes:

a noise reduction section that generates a second medical image in which noise and artifacts are reduced with respect to an original image acquired by the imaging unit;
a difference image generation section that takes a difference for each pixel between the original image and the second medical image and generates a difference image;
a weight calculation section that calculates a weighting value for each pixel by using the difference image;
a weight decision section that receives a user instruction including a change of the weighting value for each pixel and decides on a final weighting value; and

a composite image generation section that uses the weighting value decided on by the weight decision section to combine the original image and the second medical image through weighted averaging for each pixel and generates a third medical image.

**17.** An image processing method of generating and presenting a third medical image by using a first medical image acquired by a medical imaging apparatus and a second medical image obtained by performing processing of reducing noise and artifacts with respect to the first medical image, the image processing method comprising:

taking a difference for each pixel between the first medical image and the second medical image and generating a difference image;
calculating a weighting value for each pixel by using the difference image;
receiving a user instruction including a change of the weighting value for each pixel and deciding on a final weighting value; and
using the decided-on weighting value to combine the first medical image and the second medical image through weighted averaging for each pixel.

**18.** The image processing method according to claim 17,
wherein, in the calculation of the weighting value, a weight coefficient $\alpha$ with respect to a pixel value is calculated using the difference image, and the weight coefficient $\alpha$ is used as an exponent of a fixed weight W to decide on the weighting value for each pixel, which is denoted by $W^{\alpha}$.

**19.** The image processing method according to claim 17,
wherein receiving the user instruction is receiving any one of the weighting value, a weight which is a function of the weighting value and the weight coefficient, or the weight coefficient.

**20.** The image processing method according to claim 17, further comprising:

before receiving the user instruction,
generating a provisional composite image by weighted averaging the first medical image and the second medical image for each pixel using the calculated weighting value; and
displaying the provisional composite image and a display bar of a weight,
wherein the user instruction is received through an operation of the display bar.

FIG. 1

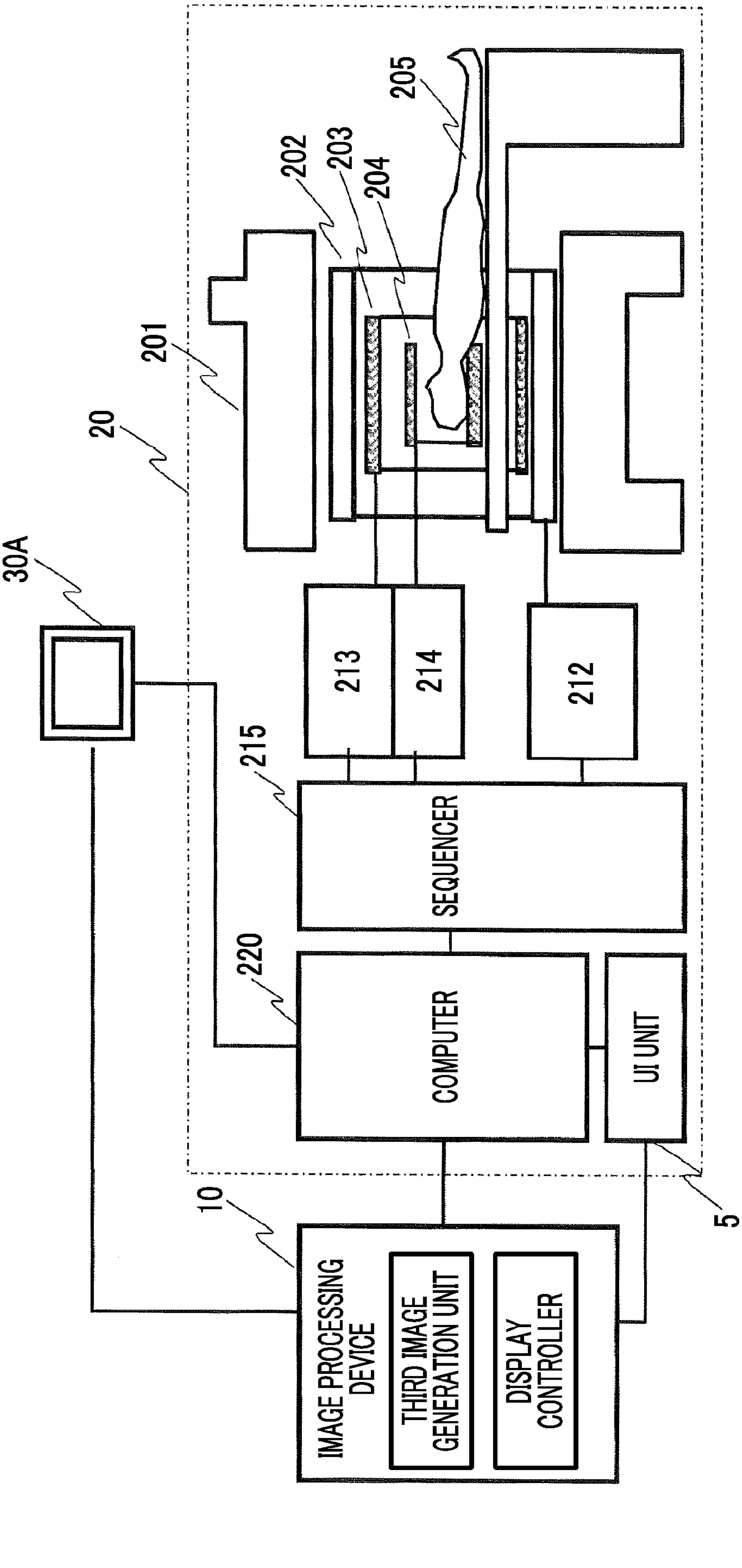
30A
20
201
202
203
204
205
213
214
212
215
SEQUENCER
220
COMPUTER
UI UNIT
5
10
IMAGE PROCESSING DEVICE
THIRD IMAGE GENERATION UNIT
DISPLAY CONTROLLER

FIG. 2

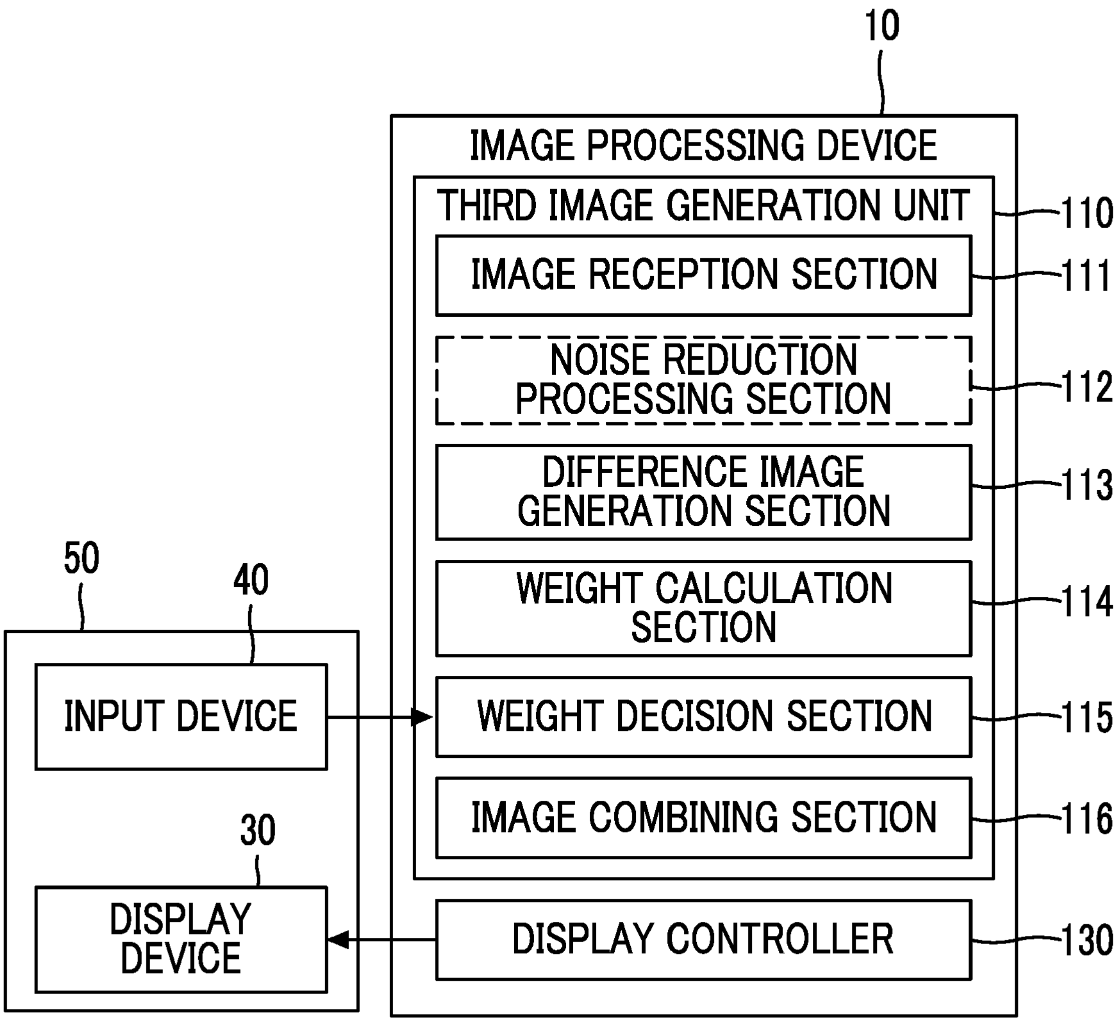
10
IMAGE PROCESSING DEVICE
THIRD IMAGE GENERATION UNIT
110
IMAGE RECEPTION SECTION
111
NOISE REDUCTION PROCESSING SECTION
112
DIFFERENCE IMAGE GENERATION SECTION
113
WEIGHT CALCULATION SECTION
114
WEIGHT DECISION SECTION
115
IMAGE COMBINING SECTION
116
DISPLAY CONTROLLER
130
50
40
INPUT DEVICE
30
DISPLAY DEVICE

FIG. 3

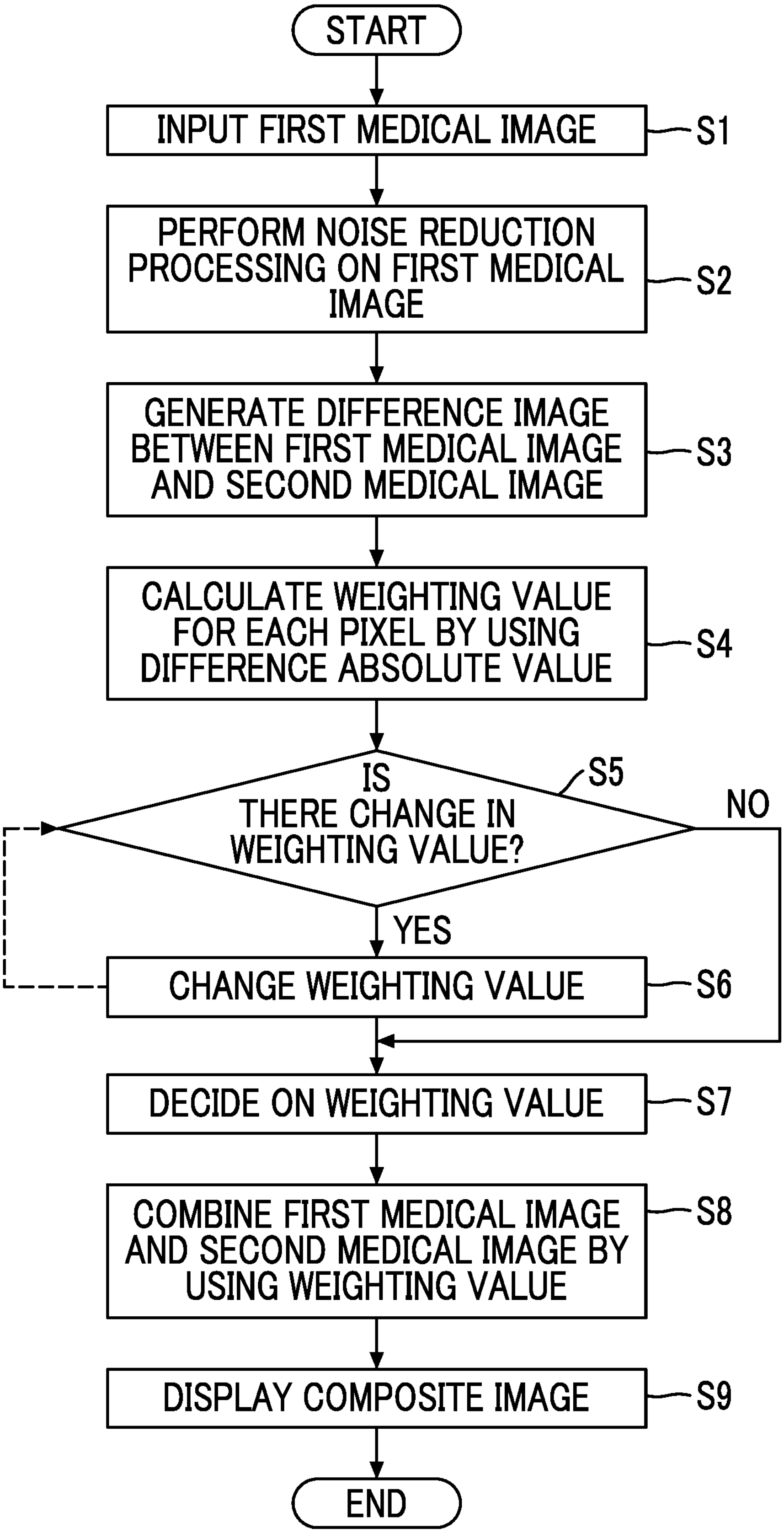
START
INPUT FIRST MEDICAL IMAGE
S1
PERFORM NOISE REDUCTION PROCESSING ON FIRST MEDICAL IMAGE
S2
GENERATE DIFFERENCE IMAGE BETWEEN FIRST MEDICAL IMAGE AND SECOND MEDICAL IMAGE
S3
CALCULATE WEIGHTING VALUE FOR EACH PIXEL BY USING DIFFERENCE ABSOLUTE VALUE
S4
IS THERE CHANGE IN WEIGHTING VALUE?
S5
NO
YES
CHANGE WEIGHTING VALUE
S6
DECIDE ON WEIGHTING VALUE
S7
COMBINE FIRST MEDICAL IMAGE AND SECOND MEDICAL IMAGE BY USING WEIGHTING VALUE
S8
DISPLAY COMPOSITE IMAGE
S9
END

FIG. 4

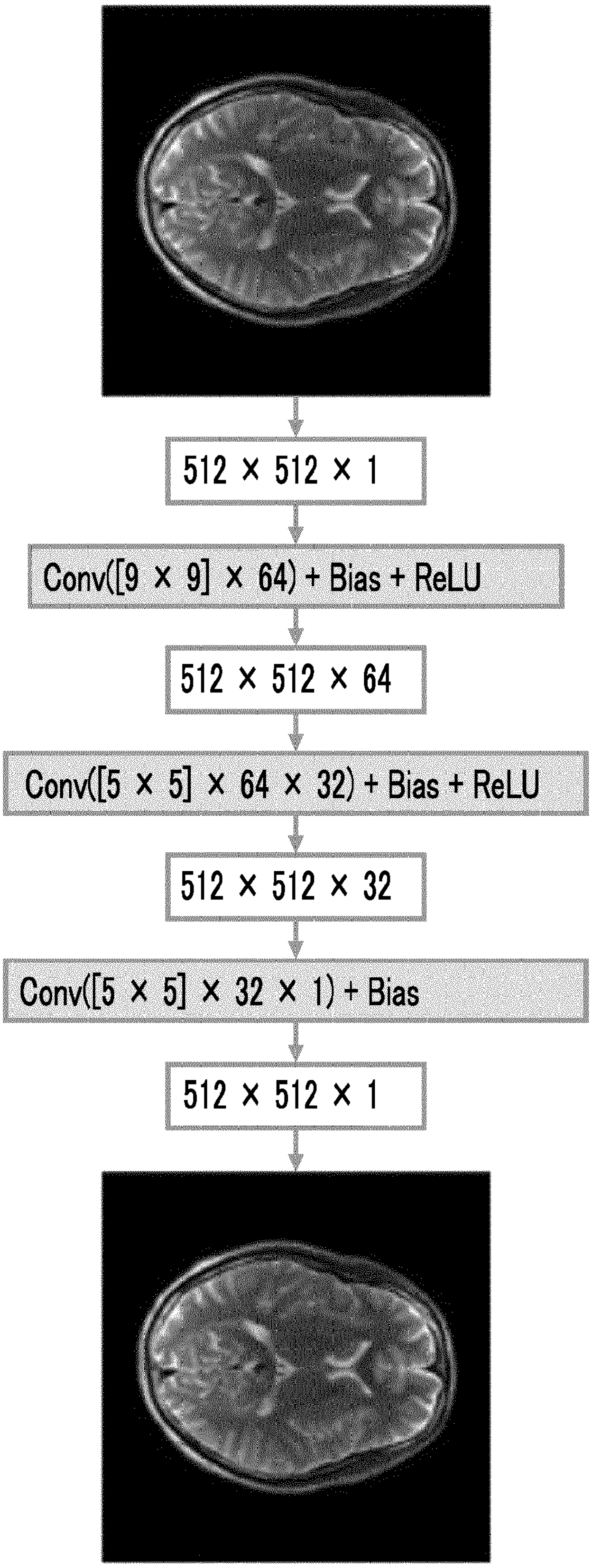
512 × 512 × 1
Conv([9 × 9] × 64) + Bias + ReLU
512 × 512 × 64
Conv([5 × 5] × 64 × 32) + Bias + ReLU
512 × 512 × 32
Conv([5 × 5] × 32 × 1) + Bias
512 × 512 × 1

FIG. 5

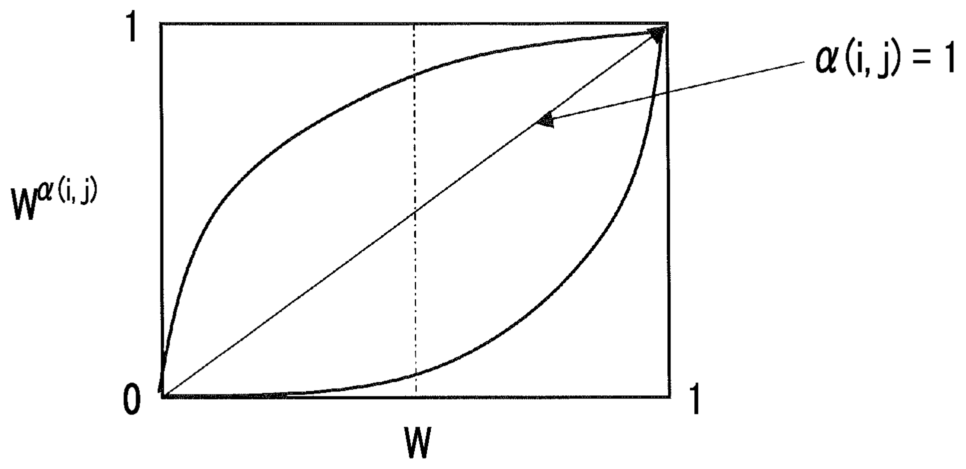


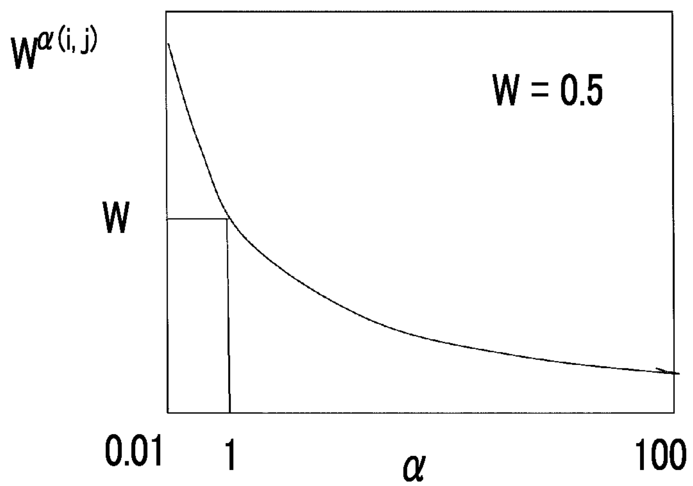

FIG. 6A

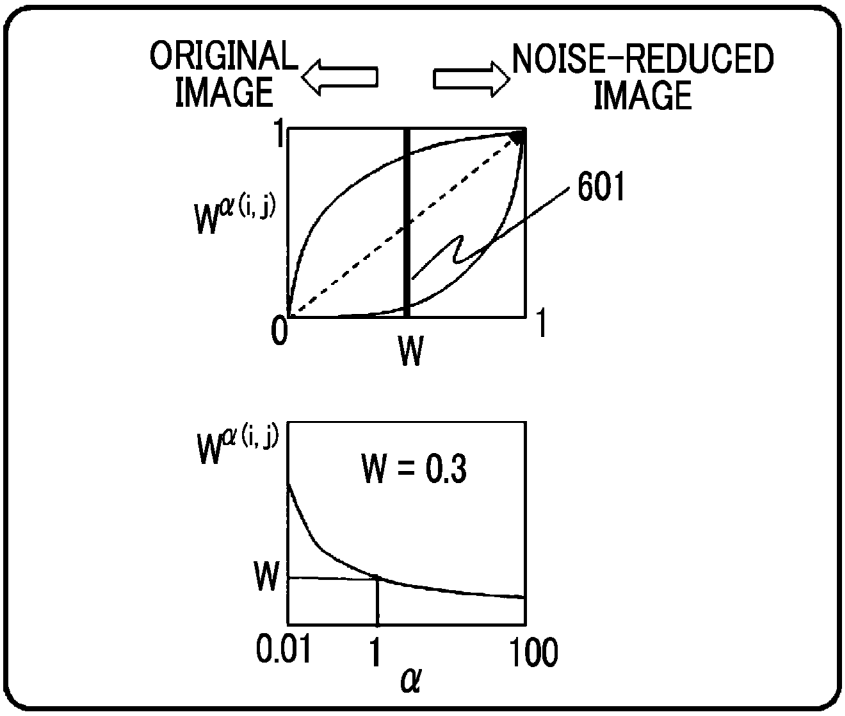
ORIGINAL IMAGE
NOISE-REDUCED IMAGE
1
$W^{\alpha(i,j)}$
601
0
W
1
$W^{\alpha(i,j)}$
W = 0.3
W
0.01
1
100
α

FIG. 6B

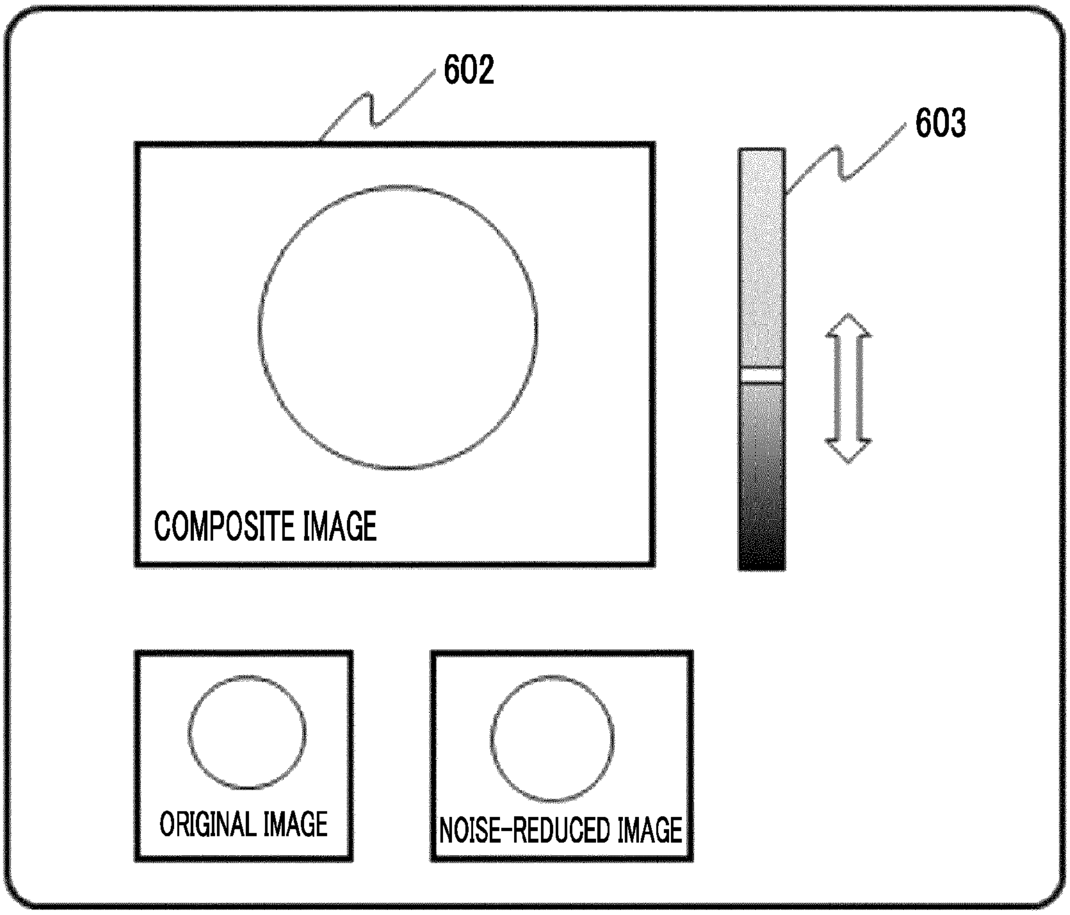
602
603
COMPOSITE IMAGE
ORIGINAL IMAGE
NOISE-REDUCED IMAGE

FIG. 7

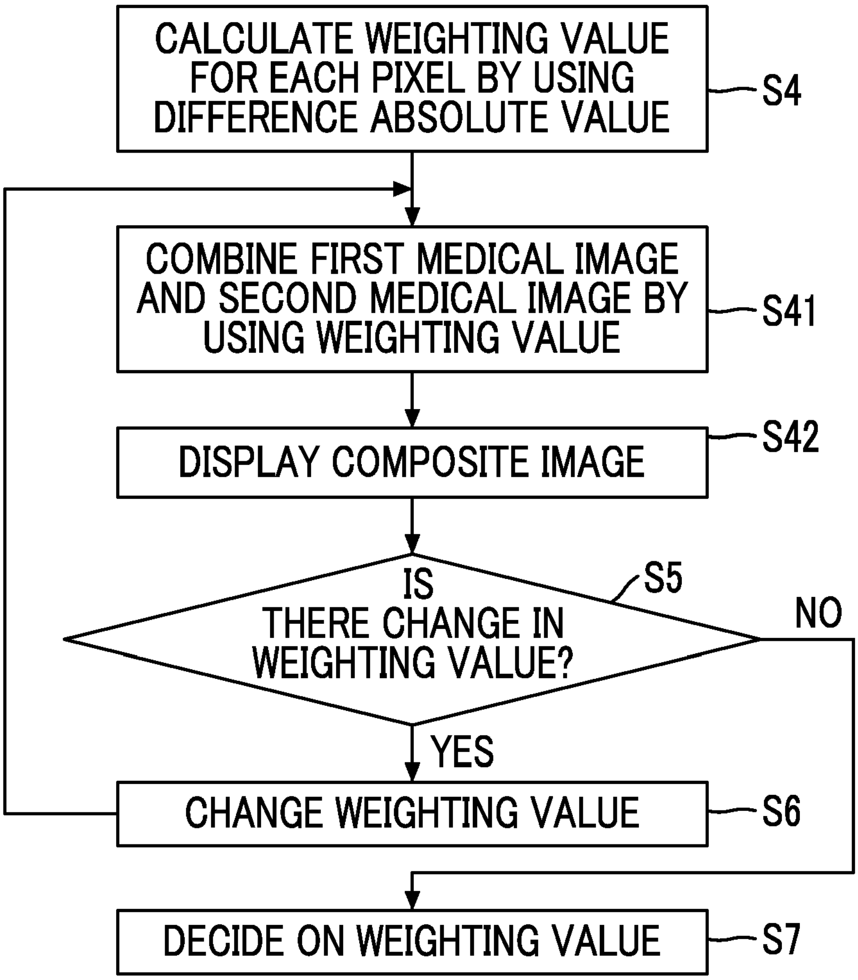
CALCULATE WEIGHTING VALUE FOR EACH PIXEL BY USING DIFFERENCE ABSOLUTE VALUE
S4
COMBINE FIRST MEDICAL IMAGE AND SECOND MEDICAL IMAGE BY USING WEIGHTING VALUE
S41
DISPLAY COMPOSITE IMAGE
S42
IS THERE CHANGE IN WEIGHTING VALUE?
S5
NO
YES
CHANGE WEIGHTING VALUE
S6
DECIDE ON WEIGHTING VALUE
S7

FIG. 8

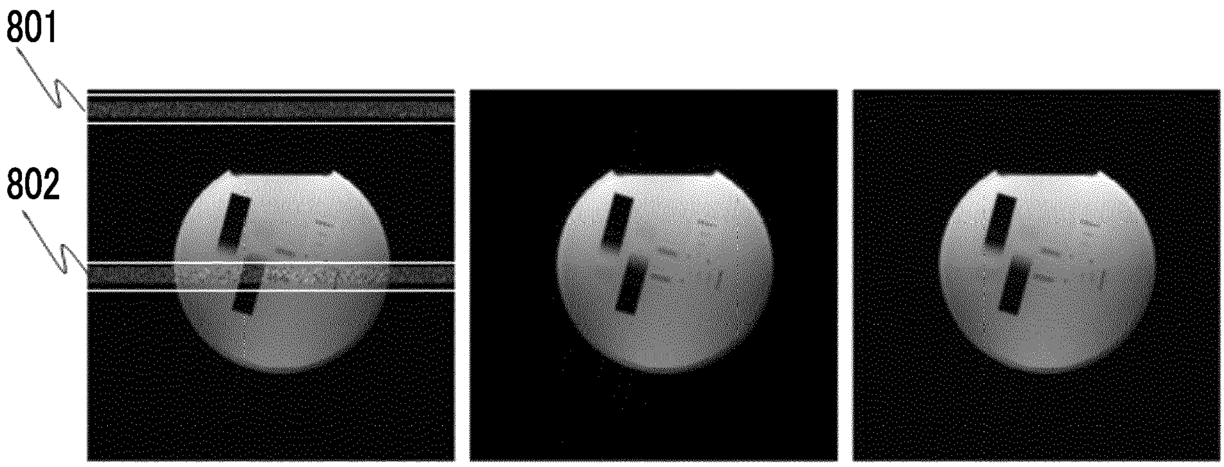


$I_{org}(i,j)$
IMAGE BEFORE CNN PROCESSING

$I_{CNN}(i,j)$
IMAGE AFTER CNN PROCESSING

$I_{adj}(i,j)$
ADJUSTED IMAGE

FIG. 9

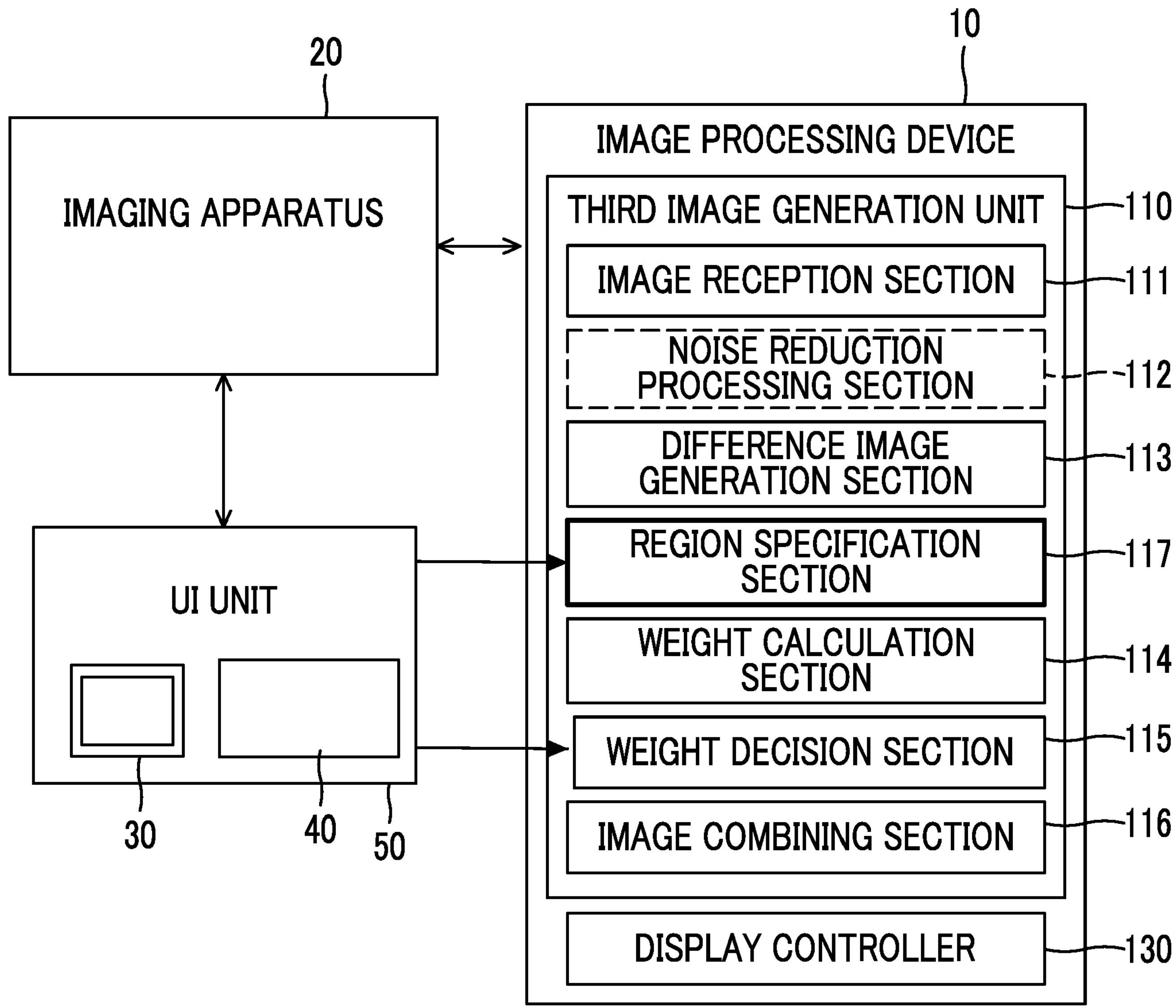
20
IMAGING APPARATUS
UI UNIT
30
40
50
10
IMAGE PROCESSING DEVICE
THIRD IMAGE GENERATION UNIT
110
IMAGE RECEPTION SECTION
111
NOISE REDUCTION PROCESSING SECTION
112
DIFFERENCE IMAGE GENERATION SECTION
113
REGION SPECIFICATION SECTION
117
WEIGHT CALCULATION SECTION
114
WEIGHT DECISION SECTION
115
IMAGE COMBINING SECTION
116
DISPLAY CONTROLLER
130

FIG. 10

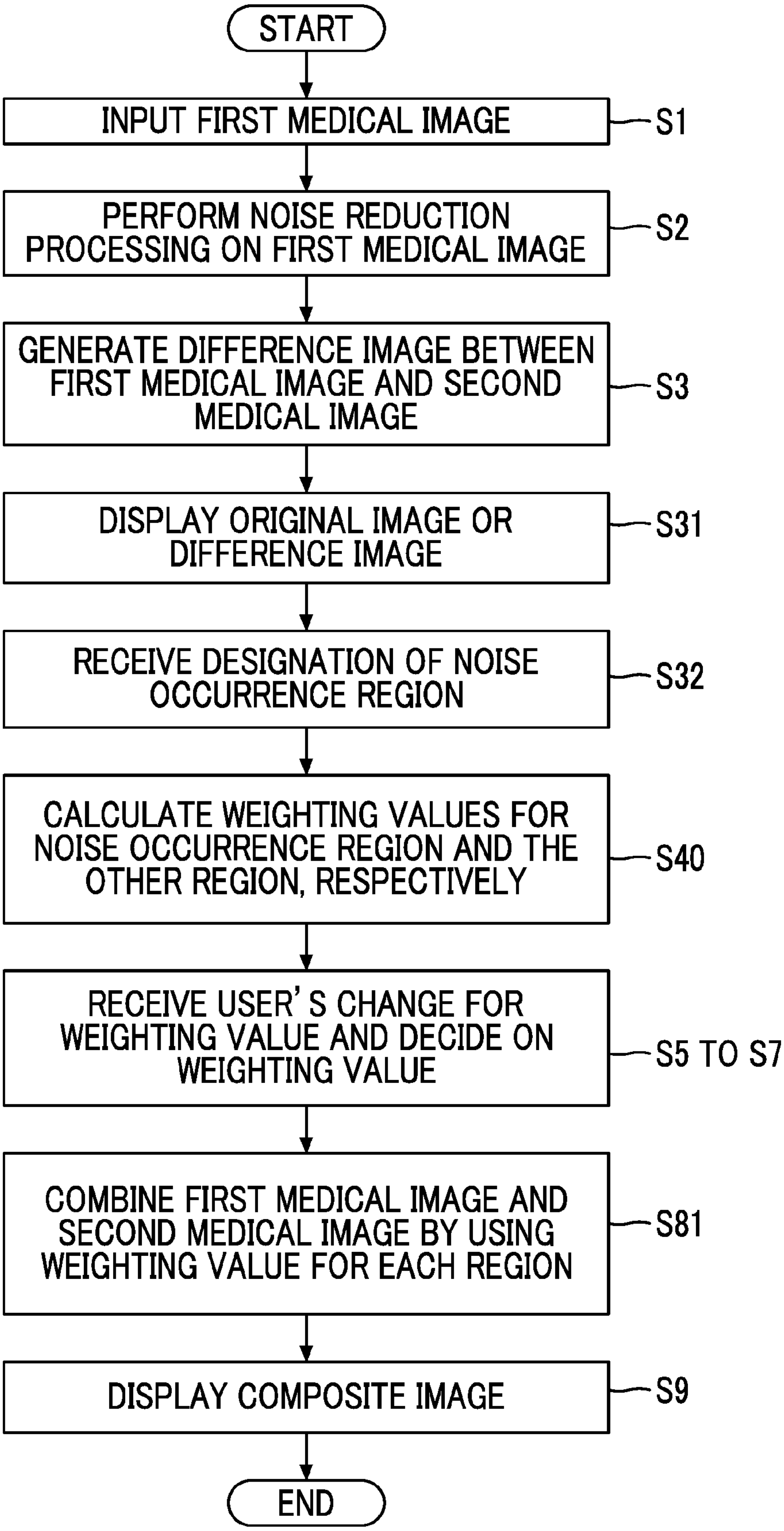
START
INPUT FIRST MEDICAL IMAGE
S1
PERFORM NOISE REDUCTION PROCESSING ON FIRST MEDICAL IMAGE
S2
GENERATE DIFFERENCE IMAGE BETWEEN FIRST MEDICAL IMAGE AND SECOND MEDICAL IMAGE
S3
DISPLAY ORIGINAL IMAGE OR DIFFERENCE IMAGE
S31
RECEIVE DESIGNATION OF NOISE OCCURRENCE REGION
S32
CALCULATE WEIGHTING VALUES FOR NOISE OCCURRENCE REGION AND THE OTHER REGION, RESPECTIVELY
S40
RECEIVE USER'S CHANGE FOR WEIGHTING VALUE AND DECIDE ON WEIGHTING VALUE
S5 TO S7
COMBINE FIRST MEDICAL IMAGE AND SECOND MEDICAL IMAGE BY USING WEIGHTING VALUE FOR EACH REGION
S81
DISPLAY COMPOSITE IMAGE
S9
END

FIG. 11

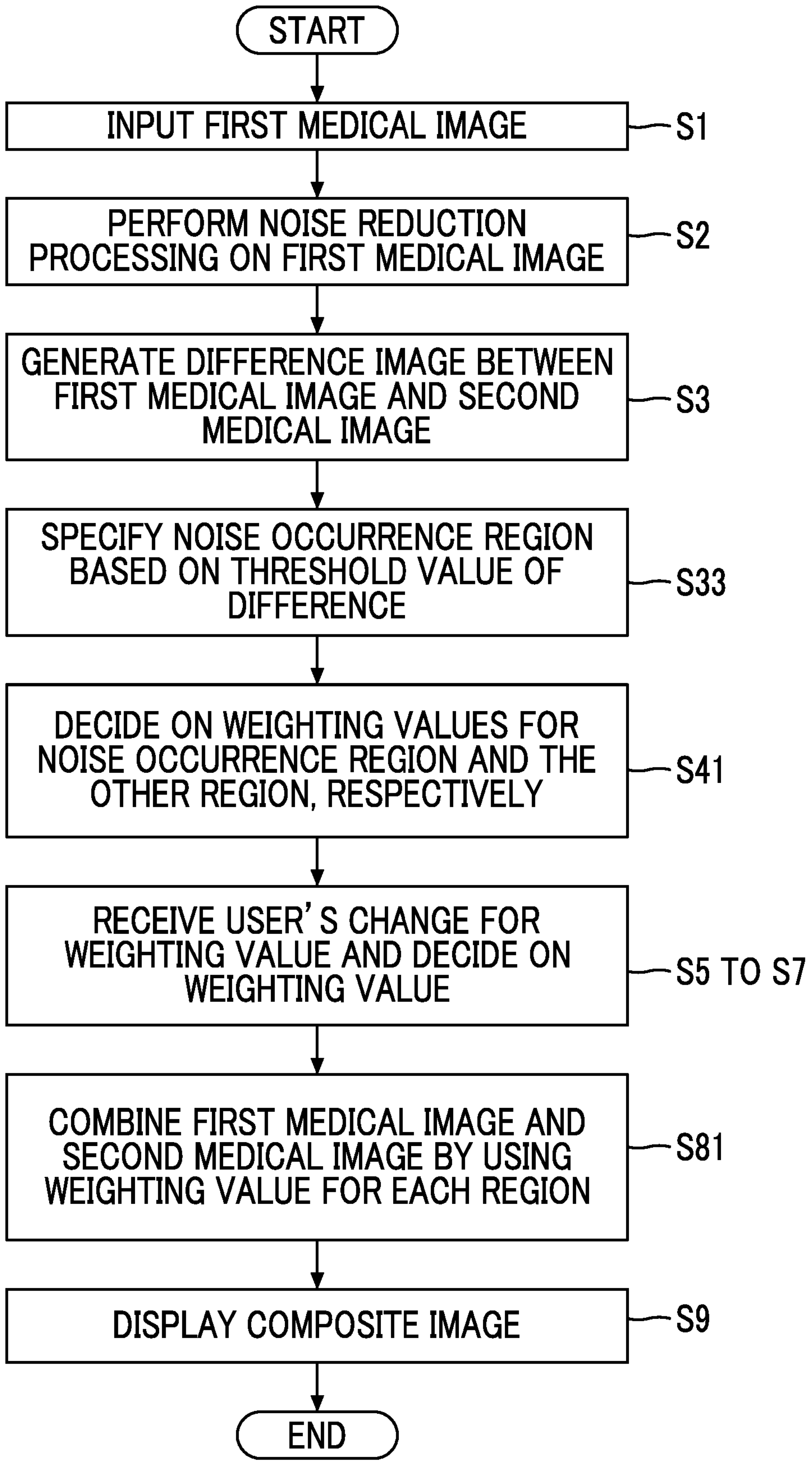
START
INPUT FIRST MEDICAL IMAGE
S1
PERFORM NOISE REDUCTION PROCESSING ON FIRST MEDICAL IMAGE
S2
GENERATE DIFFERENCE IMAGE BETWEEN FIRST MEDICAL IMAGE AND SECOND MEDICAL IMAGE
S3
SPECIFY NOISE OCCURRENCE REGION BASED ON THRESHOLD VALUE OF DIFFERENCE
S33
DECIDE ON WEIGHTING VALUES FOR NOISE OCCURRENCE REGION AND THE OTHER REGION, RESPECTIVELY
S41
RECEIVE USER'S CHANGE FOR WEIGHTING VALUE AND DECIDE ON WEIGHTING VALUE
S5 TO S7
COMBINE FIRST MEDICAL IMAGE AND SECOND MEDICAL IMAGE BY USING WEIGHTING VALUE FOR EACH REGION
S81
DISPLAY COMPOSITE IMAGE
S9
END

FIG. 12

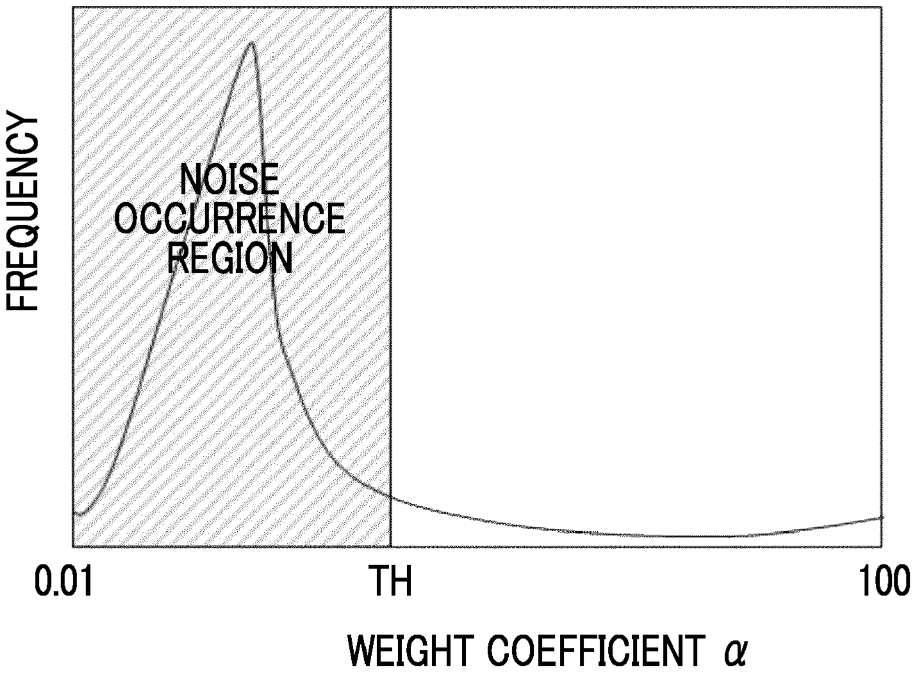
FREQUENCY
NOISE
OCCURRENCE
REGION
0.01
TH
100
WEIGHT COEFFICIENT $\alpha$

FIG. 13

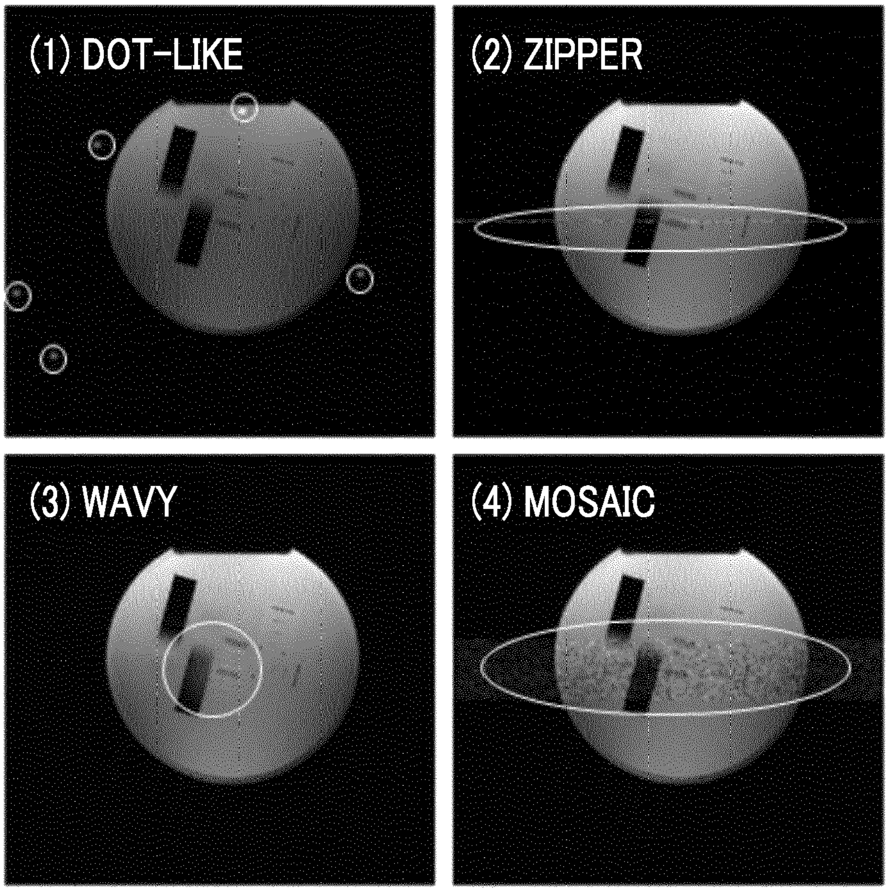

FIG. 14

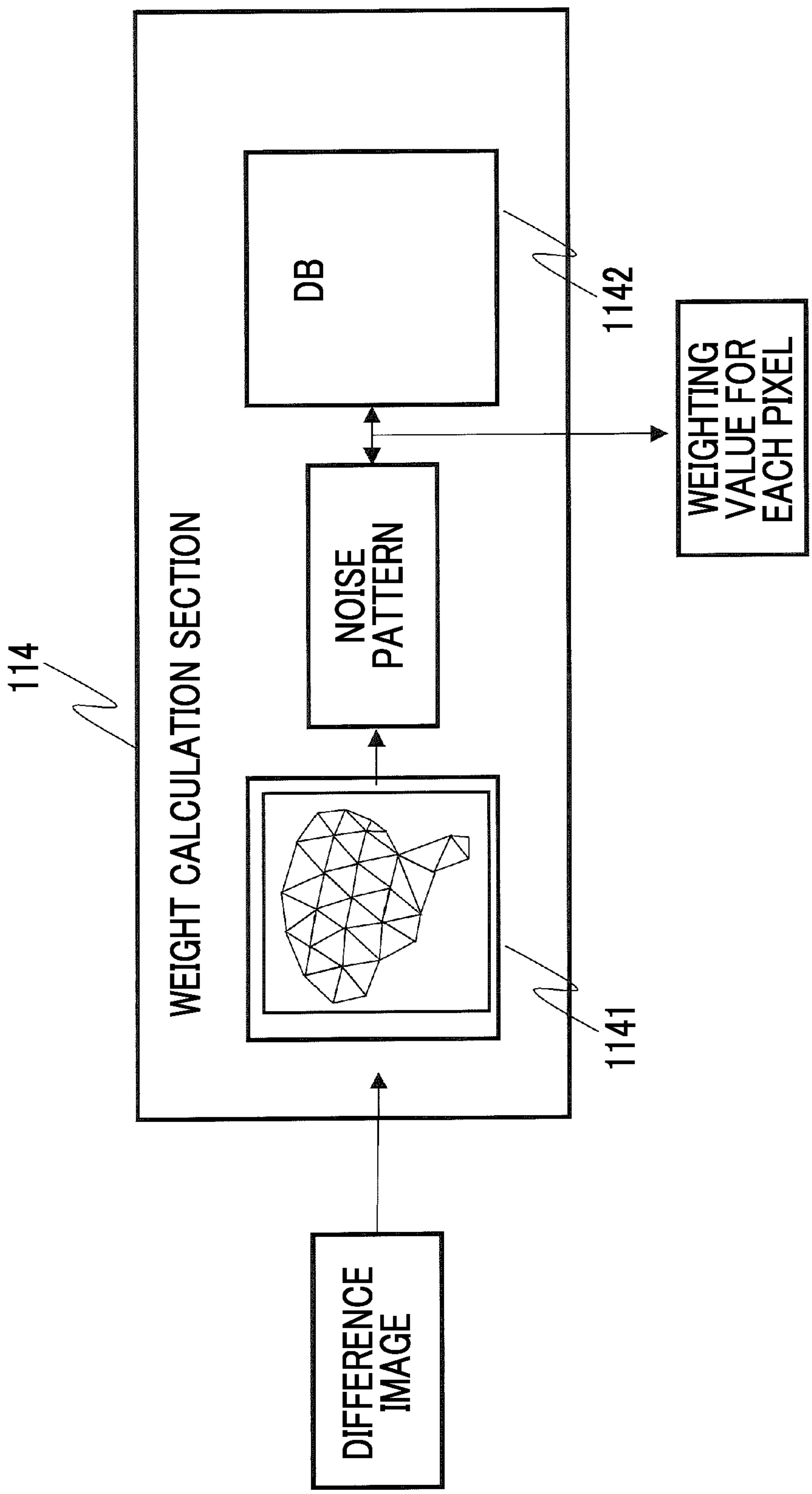

114
WEIGHT CALCULATION SECTION
DB
1142
NOISE PATTERN
WEIGHTING VALUE FOR EACH PIXEL
1141
DIFFERENCE IMAGE

FIG. 15

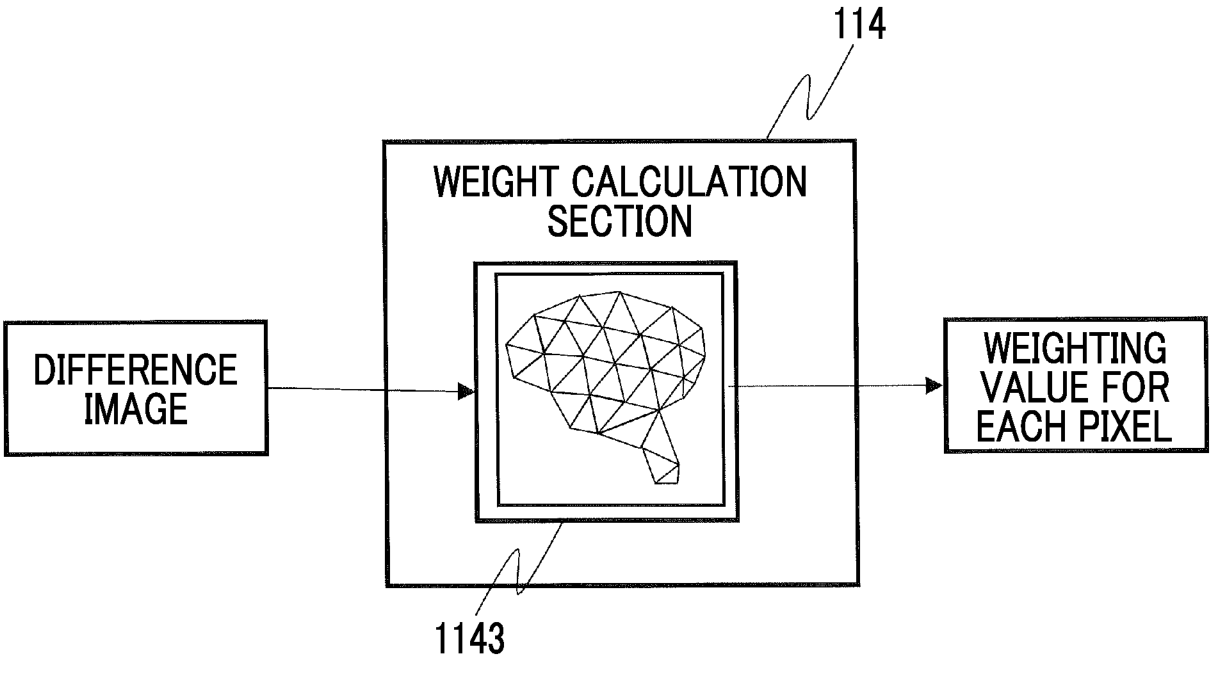
114
WEIGHT CALCULATION SECTION
DIFFERENCE IMAGE
WEIGHTING VALUE FOR EACH PIXEL
1143

Europäisches Patentamt
European Patent Office
Office européen des brevets

# EUROPEAN SEARCH REPORT

Application Number
EP 24 15 4613

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 1 447 773 A2 (TOSHIBA KK [JP]; TOSHIBA MEDICAL SYS CORP [JP]) 18 August 2004 (2004-08-18) * paragraphs [0001], [0009] * * figure 1 * * paragraphs [0043], [0050], [0061] * ----- | 1-20 | INV. G06T5/20 G06T5/70 G06T5/75 G06T5/60 |
| A | US 2014/211034 A1 (TANAKA KOICHI [JP]) 31 July 2014 (2014-07-31) * figure 13 * * paragraphs [0126] - [128,] * * abstract * ----- | 1-20 | |
| A | US 2008/095462 A1 (HSIEH JIANG [US] ET AL) 24 April 2008 (2008-04-24) * paragraphs [0035], [0033], [0036] * ----- | 1-20 | |
| | | | TECHNICAL FIELDS SEARCHED (IPC) |
| | | | G06T |

The present search report has been drawn up for all claims

2

| Place of search | Date of completion of the search | Examiner |
|---|---|---|
| Munich | 11 June 2024 | Winkler, Gregor |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 24 15 4613

This annex lists the patent family members relating to the patent documents cited in the above-mentioned European search report. The members are as contained in the European Patent Office EDP file on
The European Patent Office is in no way liable for these particulars which are merely given for the purpose of information.

11-06-2024

| Patent document cited in search report | Publication date | Patent family member(s) | Publication date |
|---|---|---|---|
| EP 1447773 A2 | 18-08-2004 | CN 1521695 A | 18-08-2004 |
| | | DE 602004003845 T2 | 30-08-2007 |
| | | EP 1447773 A2 | 18-08-2004 |
| | | EP 1755082 A2 | 21-02-2007 |
| | | JP 4413504 B2 | 10-02-2010 |
| | | JP 2004246625 A | 02-09-2004 |
| | | US 2004159812 A1 | 19-08-2004 |
| US 2014211034 A1 | 31-07-2014 | CN 103843033 A | 04-06-2014 |
| | | JP 5554453 B2 | 23-07-2014 |
| | | JP WO2013047405 A1 | 26-03-2015 |
| | | US 2014211034 A1 | 31-07-2014 |
| | | WO 2013047405 A1 | 04-04-2013 |
| US 2008095462 A1 | 24-04-2008 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2023020136 A
- WO 2009128213 A
- JP 2020119429 A